# EUROPEAN PATENT APPLICATION

(11) **EP 2 789 295 A1**
(43) Date of publication of application: **15.10.2014**
(21) Application number: 13163524.5
(22) Date of filing: 12.04.2013
(51) Int. Cl.: A61B 5/145, G06Q 30/02

(54) **Improved measurement device, system and method**

(71) Applicant: Modz Oy, 00810 Helsinki (FI)
(72) Inventor: Koski, Salla, 00810 Helsinki (FI); Tasanen, Mikko, 00810 Helsinki (FI); Rajala, Carina, 00810 Helsinki (FI)
(74) Representative: Kupiainen, Juhani Kalervo

(57) **Abstract**

The object of the invention is a measurement device and a system for measuring and monitoring information which relates to diabetes care. The purpose of the invention is to motivate a user to perform activities that are required in the care. In a preferred embodiment of the invention a performance levels are determined and the level of a user is based on the activities and/or measurement results of the user. Upon increase in the user's level the user is awarded with e.g. new reminder figure in the device. The motivation of a user to follow care activities is thus increased.

## Description

### Field of invention

An object of the invention is a measurement device, a system and a method for measuring and monitoring data which relates to human health. The invention is especially applicable for e.g. monitoring and care of diabetes disease.

### Background technology

A person with a diabetes disease must take especially good care for his/her diet, general condition, regular rest and medication. The care of the diabetes disease also demands regular concentration measurement of blood sugar i.e. blood glucose. The measurement must be performed even several times a day. The measurement commonly takes place by taking a small sample of blood from a finger tip, which sample is let to absorb into a sample test dot of a sample test strip. The glucose concentration is measured from the strip with a portable measurement device, for example. The sample test strip is placed to the measurement device, after which the measurement device performs blood glucose measurement from a blood sample situated at the sample test dot, and shows the measurement result on the digital display of the measurement device. When a person knows the glucose concentration he/she may assess, whether he/she has need for an instant care action. If the measured blood glucose concentration is too high, e.g. more than 15 mmol/l, the care action is generally adding insulin to blood by injection. If the measured blood glucose concentration is too low, e.g. less than 3 mmol/l, the care action is generally taking drink or food which contains carbon hydrate. In addition to assessing an instant care action the blood glucose concentration gives information on requirement of eating, physical exercise, rest or medication.

In addition to measurement devices of blood sugar concentration there are also programs to be installed in a personal computer, with which a person can monitor the development of blood glucose concentration. When using the program, the measured data can be input to a computer for an analysis performed by the program.

In spite of the fact that portable measurement devices and care equipment are available for caring diabetes, the monitoring and care is insufficient for a large portion of people suffering diabetes. This is caused by the following exemplary factors:
- Children and young people do not understand or remember the matters relating to diabetes care and they are not interested in them. On the other hand, parents do not have a possibility to provide persistent looking after the care of the diabetes of a child. Therefore, following a care program would also require a child's high motivation and interest in the care.
- Many users are not able to use the available measurement devices and they can possibly not assess the actions that are required based on the measurement results, or to follow the program and time table related to the care.
- The doctor taking care of a person and parents of a child do not always have enough information on realisation of the care and of the development and the variation of the glucose values.

There also exist systems, by which it is possible to transfer measurement results of the measurement devices to user's work station and possibly further to personnel of the health care, and to perform analyses on the information. Such systems give information on the history of the measurement results, but they do not enhance the motivation of a user for daily care.

Since diabetes is quite a common disease, insufficient care is a significant problem except for the person himself/herself but also for the society. Therefore, the improvement of care and solving the above problems is important.

### Summary of the invention

The object of the invention is to provide a new solution for measuring and monitoring information which relates to blood glucose concentration of a user, by which solution the above problems can be decreased or avoided. The object is therefore to provide a solution which enhances the motivation of the user to perform the determined care activities.

The object of the invention is achieved with a solution in which a portable measurement device of a user has measurement means for measuring blood glucose concentration and means for storing user specific care data. The device is also arranged to perform an award action when a user fulfils determined performance. In a preferable embodiment the device is arranged to store data on performance levels, which are compared to measurement results and activities performed by the user. The award action is made when the performance level of the user is increased to a higher level.

In one embodiment of the invention the award action is providing a new figure /sound for reminders of the device.

In one embodiment of the invention the system includes a game program which is loaded on the user device or a server, to which program the users or a certain user group has an access. A user is then awarded with some virtual advantage in the game according to the measurement results and based on how the user has followed the care plan.

A user portable measurement and monitoring device according to one aspect of the invention, which device comprises a measurement unit for measuring blood glucose concentration, and a user interface for presenting to a user information which is based on a measurement result, the user interface comprising at least one display and/or means for forming sound,
is characterised in that the measurement and monitoring device comprises
- means for storing user specific data which relates to care of the user,
- means for providing the user with reminders and/or instructions,
- means for comparing a measurement result with said care data,
- means for recording user activities,
- means for storing data on performance levels, and determining a user level on the basis of the comparison and/or the user activities, and
- means for performing an action in order to award the user on the basis of an increase on the user level.

A measurement and monitoring system according to another aspect of the invention, which system comprises a server and a portable measurement device, the measurement device further comprising a measurement unit for measuring blood glucose concentration and a user interface for presenting to a user information which is based on a measurement result, the user interface comprising at least one display and/or means for forming sound, and the system comprising means for communication between the measurement device and the server,
is characterised in that the system comprises
- means for storing user specific data which relates to care of the user,
- means for providing the user with reminders and/or instructions,
- means for comparing a measurement result with said care data,
- means for recording user activities,
- means for communicating measurement results, user specific data and/or activity data between the measurement device and the server,
- means for storing data on performance levels, and determining a user level on the basis of the comparison and/or the user activities, and
- means for performing an action in order to award the user on the basis of on an increase on the user level.

A method according to a further aspect of the invention for measuring and monitoring blood glucose concentration with a portable measurement device, wherein user information which is based on a measurement result is presented on a user interface by displaying and/or forming sound,
is characterised in that the method comprises
- storing user specific data which relates to care of the user,
- providing the user with reminders and/or instructions,
- comparing a measurement result with said care data,
- recording user activities,
- communicating measurement results, user specific data and/or activity data between the measurement device and the server,
- storing data on performance levels, and determining a user level on the basis of the comparison and/or the user activities, and
- performing an action in order to award the user on the basis of an increase on the user level.

Some preferable embodiments of the invention are described in dependent claims.

In this patent application "measurement device" means a device for measuring a magnitude which relates to human health, such as for measuring blood glucose concentration. However, a measurement device may also include monitoring actions of the measurement results, actions reminding a user, action equipment, sample substrates required in the measurements, etc.

"Measurement unit" means in this patent application a part of a measurement device, which has a measurement sensor and preferably means for defining a value of a measurement result on the basis of a signal received from the measurement sensor. A measurement unit may also comprise a memory and programs for monitoring and reminder functions and for possible other functions of a measurement unit.

"A sample substrate" means in this patent application e.g. a tape or strip, which has sample test dots for absorption and measurement of a blood sample.

In this patent application "a user interface" means broadly means and programs relating to the means, by which a user performs measurements, monitoring, care actions, detects measurement results, instructions or reminders or uses supplementary services.

"A user" of a measurement device means in this patent application primarily a person, whose health related values are measured with the measurement device.

### Brief description of drawings

In the following the invention is described with help of the enclosed drawings, in which:
- Figure 1: illustrates a block diagram of an exemplary measurement device according to the invention for the measurement and monitoring of blood glucose concentration;
- Figure 2: illustrates a perspective view of an exemplary measurement device according to the invention;
- Figure 3: illustrates a block diagram of an exemplary system according to the invention for measurement and monitoring of blood glucose concentration;
- Figures 4a and 4b: illustrate a flow diagram of an exemplary use of a measurement device according to the invention;
- Figure 5: illustrates a flow diagram of activity update and awarding action.

### Detailed description of exemplary embodiments

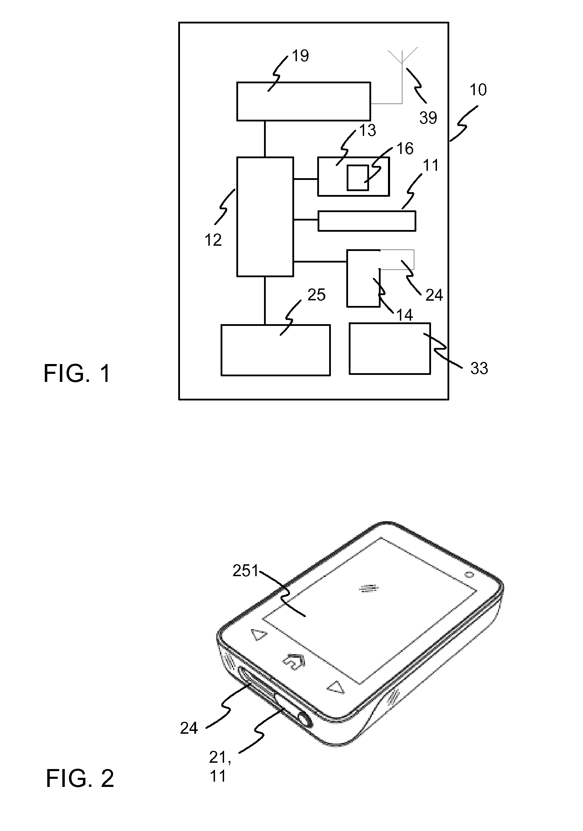

Figure 1 illustrates a block diagram of an exemplary measurement device according to the invention. The measurement device 10 includes a measurement unit 14 for measuring a blood sample. In the measurement device illustrated in Figure 1 the measurement unit 14 measures blood glucose concentration from a blood sample which is absorbed into a sample test dot. The measurement unit has an input aperture 24 for placing a test strip, on which a blood sample is then absorbed. The measurement unit is preferably based on electrical charge measurement in a manner, which is prior known as such. The measurement unit preferably includes an amplifier, an analogue-digital converter and other required electronics so that the signal received from the sensor can be fed to the input of a processor 12 of the device. The processor preferably saves the measurement results into a memory 13 for later use. The measurement device may have a single processor, or it may have two or several processor, such as a main processor and an auxiliary processor. In this case the measurement can be performed using an auxiliary processor, which transfers the data to the main processor. The main processor then handles storing and displaying the data.

Also programs 16 controlling the processor have been stored into the memory 13 of the measurement device. Further, data relating to the care program of the user is stored, which data is preferably user specific. Especially, data relating to awarding a user can be stored in the memory 13. Such data may include performance level steps, number of current user performance points and current level as well as information on awards, such as reminder Figures and sounds of each level.

The measurement device also has user interface means 25, which preferably comprise a display, such as a touch screen. Measurement results can be displayed on the touch screen in text/numbers and/or illustrated with avatars or symbols, for example. The user interface of the measurement device preferably also has means for producing voice and/or vibration. They may produce tones or speech, by means of which the user is guided and given information. The audio signals corresponding to tones or speech can be preferably formed in the processor by means of data stored in the memory. It is also possible that a user can store the figures/sounds used by the device in different performance levels. The user interface may also include other input means, such as press button switches, in addition to the touch screen input.

The measurement device has data transfer port 11, by means of which it is possible to transfer data between the measurement unit and a computer or other equipment of a user. Through the data connection it is possible to transfer measurement results and other user data which are stored by the measurement device to a terminal equipment and/or to transfer programs or user data from the computer to the measurement device. The data transfer takes place in a wired manner, e.g. via a micro-USB (Universal Serial Bus) port/connector. Such an electrical connector may be used for other purposes as well, such as connecting to other measurement devices, or charging the battery of the device with a charger or from a USB connection of a computer, for example. It may also be possible that the connection 11 of the measurement device can connect to a data transfer network, whereby it is possible to transfer data with another device which is in connection via the network.

Additionally, the measurement device 10 includes preferably one or several wireless data transfer units 19. If radio data transmission is used the data transfer unit includes an antenna 39. Then the data transfer unit may be e.g. GSM 3G or 4G module of a cellular data transfer system to which a SIM card of a user may be connected. Such a unit may include a specific processor for controlling the data transfer. The communication capability in a mobile cellular system can be used, for example, for transferring measurement data and other data from a measurement device to a mobile phone. Also the data relating to awarding the user can be communicated between a server and the user device using this communication capability. If a user is a child, the parents may receive the measurement data to their mobile phones. Also, if a user is an elderly person the relatives of the person may receive the measurement data.

The measurement device may also have a communication unit for Bluetooth communication, for example. Such wireless communication can be used with other measurement devices, such as a device measuring movement of the user or a device measuring heart pulse in order to receive other measurement data. This other measurement data can then be used as a further input in user's care program and it can also be used as the basis of performance levels and awards. However, it may be possible to connect such devices to the wired electrical connection 11 as well.

The data transfer means 11 and 19 may also transfer data between the measurement device and care devices. For example, a measurement device can transmit measurement data to an insulin injector and/or receive from an insulin injector information that the user has received an insulin dose. The measurement device may also use this information as an acknowledgement for the given activity instruction and as information which affects the reminders and activity instructions according to the care program of the user.

As described above, the measurement device may give reminders for the user. The time table for providing the reminders as well as figures and sounds for providing reminders are stored in the memory 13 of the measurement device. The reminders may relate to performing a blood sample measurement, taking a dose of insulin, having a meal, having physical exercise, and resting, for example. The figures and/or sounds of such reminders are preferably related to the performance level of the user. A user may also have a possibility to store such figures/sounds to be used on selected levels.

Based on the measurement results, the measurement device may give an instruction for eating carbon hydrates or taking a dose of insulin. The processor 12 performs the appropriate reminders according to the stored time table. The user acknowledges the reminders and instructions with the corresponding input at the touch screen of the device, for example.

The device of Figure 1 also includes an energy source 33, such as a rechargeable or disposable battery. A rechargeable battery may be charged via the USB connection, for example.

Figure 2 illustrates a perspective view of an exemplary measurement device according to the invention. The measurement device has a large touch screen display 175, which displays the measurement result and possibly other reminders or activity instructions. It preferably shows the measurement result, reminders and/or other activity instructions as figures such as avatars. The device preferably also has audio means for producing sounds, voices and/or melodies. A sample input 24 and a USB connector 11 are located at the bottom side of the device. There is a sliding cover 21, which can be moved to cover either the sample input 24 or the electrical connection 11.

Table 1 illustrates some examples of symbols, which can be presented on the display of the measurement device. The first symbol illustrates a drop, which drop means a reminder for measuring blood glucose concentration. The second symbol includes Z characters, which means an activity instruction to sleep. In the third symbol a fork is shown, which means an activity instruction for eating. The fourth symbol shows an insulin injector, which means an activity instruction for injecting insulin. The fifth symbol shows feet, reminding of physical exercise. The next four symbols inform or remind of various activities of the device.

The last five symbols of the table inform a result of a blood glucose measurement. The symbol in the middle shows a smile expression, which means that the result of the blood glucose concentration measurement has been good. The firs and last ones of those symbols mean that the result of the blood glucose concentration measurement has been poor because the sugar concentration of blood has been too low or too high, respectively. The symbols between the good and poor mean that the measurement result has been moderate/low or moderate high, respectively. This kind of measurement result may mean at the same time an activity instruction to perform the measurement again after a predetermined period of time.

It should be noted that the symbols of table 1 are only shown as examples. Instead of symbols, it is also possible to show text or figures such as avatars. The measurement results are well visualized by using certain defined colours. For example, a poor measurement result due to too low sugar concentration value could be illustrated with red colour. A poor measurement result due to too high sugar concentration value could be illustrated with violet colour. A good value could be illustrated with green colour. Moderate low and moderate high values could be illustrated with yellow and blue colours, respectively. It is also possible to provide defined sounds in relation to each value range.

**Table 1. An example of a selection of symbols/characters that can be displayed.**

| | |
|---|---|
| 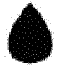 | Blood sugar measurement |
| 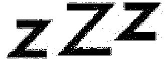 | Rest |
| 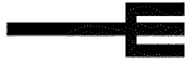 | Nutrition |
| 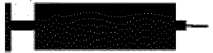 | Medication |
| 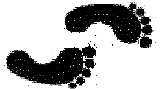 | Physical exercise |
| 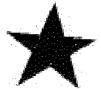 | Additional function for example, in receiving data from another device |
| 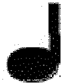 | Fanfare for good following of care program |
| 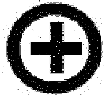 | Reminder to send measured data to the care team/contact to the care team |
| 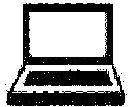 | Emptying the memory |
| 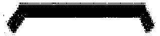 | Poor = too low blood sugar > requires actions |
| 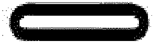 | Moderate = limit value > requires a further measurement after a moment |
| 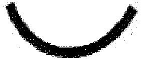 | Excellent = desired measurement result > no actions |
| 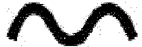 | Moderate = limit value > requires a further measurement after a moment |
| 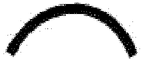 | Poor = too high blood sugar > requires actions |

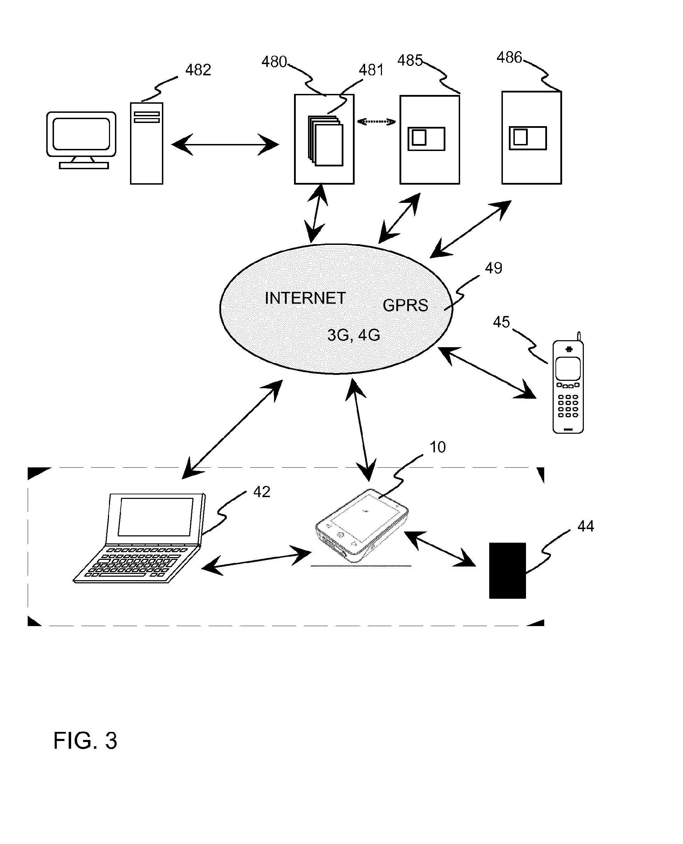

Figure 3 illustrates a system where an exemplary measurement device 10 according to the invention is in connection with several types of equipment. The measurement device 10 can be connected to a user's laptop computer 42 with a wired, USB connection, in order to transfer measurement data and other user data, as well as update programs and parameters of the measurement device. The measurement device may also be in connection with other devices 44 of the user with a wired USB connection or wireless Bluetooth or NFC connection, for example. Such other devices may include other measurement devices, an insulin injector, etc. The measurement device preferably has a wireless connection with a cellular data transfer network in order to transfer data with mobile phones 45.

The measurement device may also be in connection with a public health care system. Data is transferred with a central unit 480 of a user information centre, using direct wireless communication through cellular data network, or using a computer 42 which is connected to the Internet. The above mentioned data may then be transferred between the measurement unit and the central unit. The central unit 480 comprises a database 481, into which personal information and information relating to the disease of the measurement device users is stored. The central unit can be e.g. a central computer of regional health care. The health care system includes terminal equipment 482 of the nursing staff, which terminal equipment is connected to the central computer 480. There may also be servers 485, 486 connected to the central computer, which servers may provide supplementary services for the users, such as games.

It is possible to provide awards for good measurement performance in such a game. The award can be points or virtual goods or abilities in the game, for example. A program stored in the server 485 functions in such a way, for example, that the target information and measurement results of the users are stored and it is possible to compete among the users about how good measurement results are in relation to own target values of each user. The program may calculate by means of targets/levels and measurement results performance points that are shown in the game, whereby the real target or measurement values are not available for other users. The program includes preferably an identification functionality, by which the users log in to use the game.

A purpose of a game server is to make the activities relating to diabetes more interesting for a young user. Based on the results achieved in the game honour mentions and prizes are preferably given based on the performance levels of the users. Also the nursing staff may take part in the guiding of the games and awarding the users.

The system may also include a chat server or other social communications. The purpose of the chat server is to assist communication of such people who have the same disease, such as diabetes. This way these people can exchange their experiences and support each other. It is also possible to show the achieved awards to other users.

The central unit 480 can also be separate from other database of the health care, and which comprises only self care information relating to a certain disease, such as information on blood glucose concentration measurement and care monitoring. Nursing staff, such as a nurse and a doctor, has access to the information of the central unit. The access may take place e.g. with terminal equipment 482 after logging in the system.

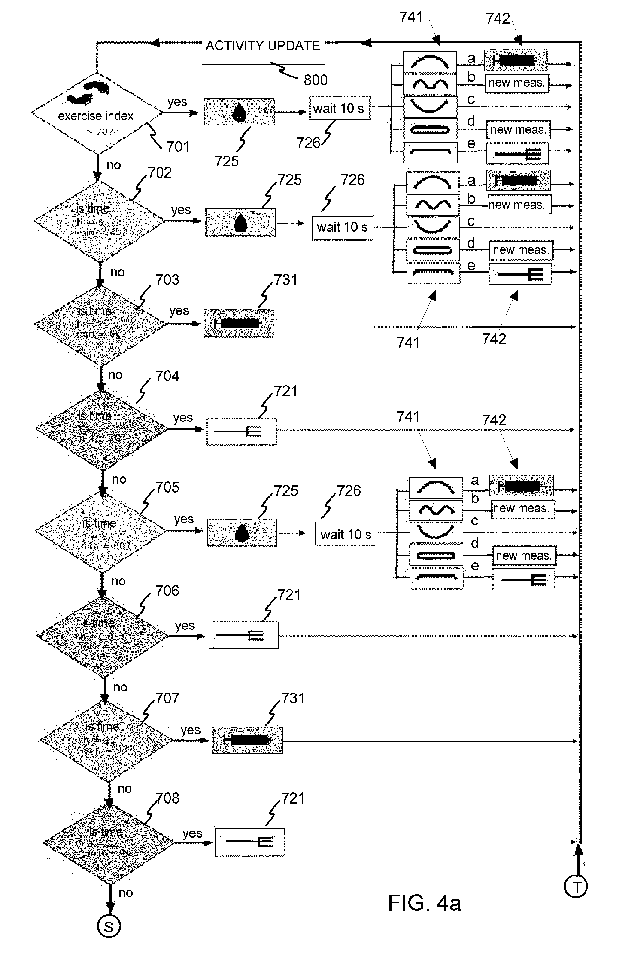

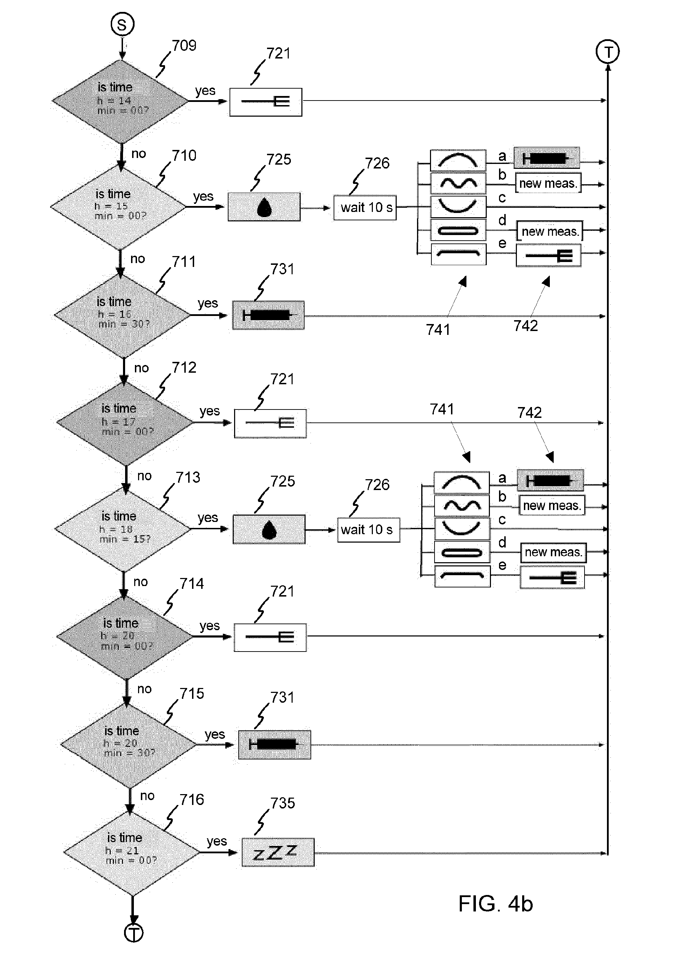

Figures 4a and 4b illustrate as a flow diagram one example of a method according to the invention. In the method of the flow diagram, the measurement device performs the checks according to the phases 701-716 repeatedly.

In the method of Figures 4a and 4b physical exercise of the user may be monitored. In phase 701 it is checked, whether the movement index of the user is higher than a predetermined limit value, such as 70. The value of the movement index can be calculated on the basis of information that user inputs to the device on physical exercise the user has performed. The value of the movement index has been transferred to the measurement device from e.g. an external device which measures the movement of the user. If the movement index exceeds the threshold value the measurement device gives an activity instruction for the user to measure sugar concentration of blood, 725. The activity instruction can be given on a display, for example, in a form of a symbol or text or in the form of tone or speech. When a sample strip has been set to the measurement device it is possibly necessary to wait for the result given by the measurement device to settle into a right value, phase 726. The length of the required waiting may be e.g. 10 s. After this, the measurement device shows the result on the display of the measurement unit as numbers and/or avatar, and/or as a sound, phase 741.

If the result of the measurement is poor, i.e. the sugar concentration of blood is too high, the measurement device gives in phase 742 an activity instruction to give an insulin dose for the user according to the alternative "a" shown in the Figure. If the measurement result is weak i.e. the sugar concentration of blood is too low, an activity instruction is given in phase 742 to eat, according to the alternative "e" shown in the Figure. If the measurement gives a limit value result the measurement device gives an activity instruction to perform the measurement again after a predetermined period of time according to alternatives "b" and "d". If the measurement result is good, the result is shown to the user, but in such a case no specific activities are necessary, alternative "c". After a possible activity relating to the measurement the user acknowledges the activity performed in phase 800.

In a method illustrated in Figures 7a and 7b the measurement device gives at predetermined points of time a reminder for measuring blood sugar concentration. Such points of time in the described example are at 6:45 o'clock, which reminder is given in phase 702, at 8.00 o'clock in phase 705, at 15:00 in phase 710 and at 18:15 in phase 713. The activity reminder can be given on a display, for example, in a form of a symbol or text, or as audio in the form of tone or speech. It is preferable that the form of the reminder depends of the performance level of the user, while a user can be awarded with a new figure/sound upon getting on a higher performance level.

When a sample strip has been set to the measurement device it is possibly necessary to wait for the result given by the measurement device to settle into a right value, phase 726. The length of the required waiting may be e.g. 10 s. After this, the measurement device shows the result on the display of the measurement unit as numbers and/or avatar, and/or as a sound, phase 741. If the result of the measurement is poor, i.e. the sugar concentration of blood is too high, the measurement device gives in phase 742 an activity instruction to give an insulin dose for the user according to the alternative "a" shown in the Figure. If the measurement result is weak i.e. the sugar concentration of blood is too low, an activity instruction is given in phase 742 to eat, according to the alternative "e" shown in the Figure. If the measurement gives a limit value result the measurement device gives an activity instruction to perform the measurement again after a predetermined period of time according to alternatives "b" and "d". If the measurement result is good, the result is shown to the user, but in such a case no specific activities are necessary, alternative "c". After a possible activity relating to the measurement the user acknowledges the activity performed in phase 800. After a possible activity relating to the measurement the user acknowledges the activity performed in phase 800.

Additionally, the measurement device has been programmed to remind giving insulin 731 to the user at predetermined points of time. In the method illustrated in the flow diagram this reminder is given at 7:00 in phase 703, at 11:30 in phase 707, et 16:30 in phase 711 and at 20:30 in phase 715. After the reminder an insulin dose is given to the user, and the user acknowledges the activity performed in phase 800.

Further, the measurement device has been programmed to remind about eating 721 at predetermined points of time. In the method illustrated in the flow diagram this reminder is given at 7:30 in phase 704, at 10:00 in phase 706, at 12:00 in phase 708, at 14:00 in phase 709, at 17:00 in phase 712, and at 20:00 in phase 714. After eating the user acknowledges the reminder in phase 800.

Additionally the measurement device reminds the user of rest, 736, at predetermined points of time. In the method illustrated in the flow diagram this reminder takes place at 21:00, phase 716. The user may input information on the amount of applied rest.

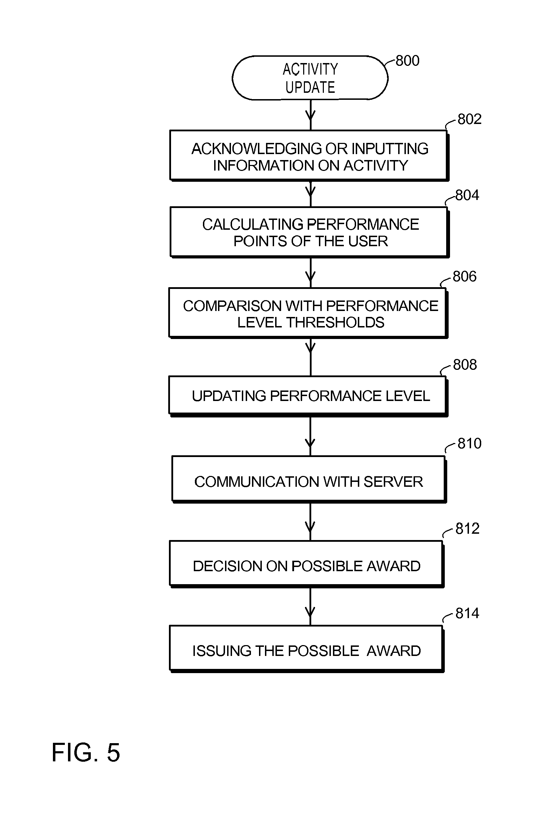

Figure 5 illustrates a method according to an embodiment of the invention for updating activities and awarding the user. This method is cited by phase 800 in Figure 4a. An activity means performing a blood glucose measurement, injecting insulin, eating, resting or performing physical exercise, for example. Such activity may be based on a reminder of the measurement device of the user. In this case the user acknowledges performing the activity. It is also possible that the user performs the activity without a reminder. In this case the user inputs information on the performed activity. The measurement device thus receives the information on the performed activity in phase 802.

It is also possible that the device suggests inputs for a user. For example, when a user makes a measurement the device can suggest whether the measurement was performed before or after a meal, and which meal of the day. The suggestion can be based on the time of the day and stored data on average time of different types of meals.

Based on the performed activity and measurement results the user device calculates performance points of the user in phase 804. The performance point value is compared to threshold values / ranges of performance levels, phase 806. The performance level of the user is then updated so that it corresponds to the current performance point value in phase 808. However, it is possible that level can only be changed higher and not lower. It is also possible that a user is first informed on the reduction of the performance points, and the level is lowered at a later stage unless the user can increase the performance point value back to the current level.

The updated performance points and/or level can be communicated to the server, phase 810. If the user level has been increased the user may get an award. Decision on issuing an award is made in phase 812. The decision can be made in the user device, automatically based on predetermined rules. For example, the user may get a new reminder Figure for the user device upon the increase of the level. It is also possible that the award decision is made in the server, especially if the award relates to server activities, such as a server game, or external prizes, such as a shopping coupon.

If it is decided to give an award to the user, the award is issued. This may mean that a reminder Figure of the user device is changed to new one. It may mean that a virtual award is given for the user in a game that is played on the user device or a server. It may also mean that a shopping coupon is mailed to the user. However, these are just a few possible examples on an award action that can be made.

One possible way to determine the performance levels is next described. On level 1 a user gets a point when the user has obtained one good measurement result. On level 2 a user gets a point when the user has obtained two good measurement results in succession. On level 3 a user gets a point when the user has obtained three good measurement results in succession. On level 4 a user gets a point when the user has obtained four good measurement results in succession. A user does not get a point upon a poor measurement. It is also possible that a point is reduced upon a poor measurement result. If a measurement is not performed in spite of a reminder, one point is reduced. A user can get to the next level when ten points are collected at a current level. Upon increase in the user's level the user is awarded with e.g. a new reminder figure and/or sound.

Above some systems and devices according to the invention have been described. The functionality according to the invention is achieved with, in addition to the devices mentioned herein, by storing the programs which relate to the inventive functions and which control the processor/processors into the memories of the devices of the system. Programming a server, terminal equipment personal computers and measurement devices is known as such for a person skilled in the art, and he/she can implement the functions of the present invention on the basis of the description given here.

It must be noted that above only some embodiments of the solution according to the invention have been described. The principle of the invention can naturally be modified within the scope of protection determined by the patent claims, e.g. in details of implementation and areas of use.

The present invention is neither restricted to use of displays, push buttons, data transfer types or functions which were described in the above embodiments, but a person skilled in the art can design several alternative implementations in the frame of the inventive features.

## Claims

1. A user portable measurement and monitoring device, which comprises a measurement unit for measuring blood glucose concentration, and a user interface for presenting to a user information which is based on a measurement result, the user interface comprising at least one display and/or means for forming sound,
**characterised in that** the measurement and monitoring device comprises
- means for storing user specific data which relates to care of the user,
- means for providing the user with reminders and/or instructions,
- means for comparing a measurement result with said care data,
- means for recording user activities,
- means for storing data on performance levels, and determining a user level on the basis of the comparison and/or the user activities, and
- means for performing an action in order to award the user on the basis of an increase on the user level.

2. The device according to claim 1, **characterised in that** the user activity relates to blood glucose measurement, insulin injection, meal, rest and/or physical exercise.

3. The device according to claim 1, **characterised in that** the award action comprises sending the measurement data and/or activity data from the user device to a server for providing an award.

4. The device according to claim 1, **characterised in that** the award action comprises determining a figure and/or sound to be used upon said reminders.

5. The device according to claim 1, **characterised in that** the figure and/or sound for a certain level is arranged to be stored by the user.

6. The device according to claim 1, **characterised in that** the award action comprises an advantage issued for a game playable in the user device or in a server.

7. The device according to claim 1, **characterised in that** the award action comprises sending determined goods for the user.

8. A measurement and monitoring system, which comprises a server and a portable measurement device, the measurement device further comprising a measurement unit for measuring blood glucose concentration and a user interface for presenting to a user information which is based on a measurement result, the user interface comprising at least one display and/or means for forming sound, and the system comprising means for communication between the measurement device and the server,
**characterised in that** the system comprises
- means for storing user specific data which relates to care of the user,
- means for providing the user with reminders and/or instructions,
- means for comparing a measurement result with said care data,
- means for recording user activities,
- means for communicating measurement results, user specific data and/or activity data between the measurement device and the server,
- means for storing data on performance levels, and determining a user level on the basis of the comparison and/or the user activities, and
- means for performing an action in order to award the user on the basis of on an increase on the user level.

9. The system according to claim 8, **characterised in that** the user activity relates to blood glucose measurement, insulin injection, meal, rest and/or physical exercise.

10. The system according to claim 8, **characterised in that** the means for providing an award for the user is comprised in the measurement device and/or in the server.

11. The system according to claim 8, **characterised in that** the award action comprises determining a figure and/or sound to be used upon said reminders.

12. The device according to claim 11, **characterised in that** the figure and/or sound for a certain level is arranged to be stored by the user into the measurement device or into the server.

13. A method measuring and monitoring blood glucose concentration with a portable measurement device, wherein user information which is based on a measurement result is presented on a user interface by displaying and/or forming sound,
**characterised in that** the method comprises
- storing user specific data which relates to care of the user,
- providing the user with reminders and/or instructions,
- comparing a measurement result with said care data,
- recording user activities,
- communicating measurement results, user specific data and/or activity data between the measurement device and the server,
- storing data on performance levels, and determining a user level on the basis of the comparison and/or the user activities, and
- performing an action in order to award the user on the basis of an increase on the user level.

14. The method according to claim 13, **characterised in that** the award action comprises determining a figure and/or sound to be used upon said reminders.

15. The method according to claim 14, **characterised in that** the figure and/or sound for a certain level is stored by the user into the measurement device or the server.
